# EUROPEAN PATENT SPECIFICATION

(11) **EP 0 709 083 B1**
(45) Date of publication and mention of the grant of the patent: **11.09.2002**
(21) Application number: 95307524.9
(22) Date of filing: 24.10.1995
(51) Int. Cl.: A61K 7/48, A61K 7/02, A61K 7/021, A61K 7/032

(54) **Cosmetic compositions**
Kosmetische Präparate
Compositions cosmétiques

(30) Priority: 25.10.1994 US 328992
(43) Date of publication of application: 01.05.1996
(73) Proprietor: Revlon Consumer Products Corporation, New York, NY 10022 (US)
(72) Inventor: Brieva, Hernando, Manalapan, New Jersey 07726 (US); Russ, Julio Gans, Westfield, New Jersey 07090 (US); Sandewicz, Ida Marie, Spotswood, New Jersey 08884 (US)
(74) Representative: Smaggasgale, Gillian Helen

(56) References cited:
- FR-A- 2 707 485
- US-A- 4 119 712
- US-A- 4 837 011
- US-A- 4 983 388
- CHEMICAL ABSTRACTS, vol. 120, no. 22, 30 May 1994 Columbus, Ohio, US; abstract no. 279893h, page 624; XP002015476 & JP-A-06 024 933 (ISEHAN K.K.) 1 February 1994

## Description

The invention is in the field of cosmetic compositions for application to the skin or hair, processes for their preparation and their use.

Cosmetic compositions are generally defined as compositions suitable for application to the human body. Cosmetic compositions such as creams and lotions are used to moisturise the skin and keep it in a smooth, supple condition. Pigmented cosmetic compositions such as foundation (makeup), blusher, lipstick and eyeshadow, are used to colour the skin and lips. Since colour is one of the most important reasons for wearing cosmetics, colour-containing cosmetics must be very carefully formulated to provide maximum wear and effect.

One of the long-standing problems with cosmetics such as foundation or face makeup, lipstick, mascara and the like, is the tendency of the cosmetic to blot or transfer from the skin or lashes onto other surfaces such as glassware, silverware or clothing. This not only creates soiling, but forces the cosmetic user to reapply cosmetic at fairly short intervals.

For example, traditional makeup compositions are either water and oil emulsions containing pigments, or they can be anhydrous systems containing waxes, oils and pigments. These formulations are applied and blended into the skin to provide colour and correct skin topography to provide an even, smooth appearance. The films are simply deposited on the surface of the skin and if touched with fingers the product may transfer or become blotchy and uneven. Perspiration or sebum will break through the film and cause running or smearing. If skin comes into contact with clothing, the clothing may become soiled.

The object of this invention is to formulate a cosmetic with long-lasting adherence to skin.

Another object of the invention is to formulate a cosmetic which yields a film which is not disturbed when blotted to remove sebum or perspiration.

Another object of the invention is to formulate a cosmetic which yields a film which does not readily transfer to clothing or utensils.

Another object of the invention is to formulate a cosmetic which yields a film which exhibits reduced permeability to oil and water.

The invention is directed to a cosmetic composition in the form of a water and oil emulsion having improved transfer resistance comprising:
a) from 0.1 to 60% by weight of trimethylated silica;
b) from 0.1 to 60% by weight of a volatile solvent having a viscosity of from 0.5 to 100 mPa·s (centipoise) at 25°C;
c) from 0.1 to 60% of by weight of dimethicone and/or dimethicone copolyol; and
d) from 0.1 to 80% of a cosmetically acceptable carrier; wherein the volatile solvent comprises a volatile silicone and wherein at least a portion of the trimethylated silica and the volatile silicone are present as a pre-blended mixture.

There is also provided, in accordance with the present invention, the process of claim 15, the product of claim 16 and the use of claim 17.

The composition of the invention contains 0.1-60%, preferably 0.5-50%, more preferably 1-30% trimethylated silica (also referred to as trimethyl siloxysilicate). The silica particles preferably have an average particle size of 0.5 to 100 nanometres (millimicrons); and may be spheroidal or non-spheroidal. The trimethylated silica and at least a portion of the volatile silicone are purchased as a pre-blended mixture. Volatile silicone and trimethylated silica suitable for use in this invention can be made in accordance with US patent specification no. 4,983,388. In the preferred embodiment of the invention, a combination of trimethylated silica and volatile silicone is purchased as a blend from Dow Corning Corporation under the tradenames Dow 2-0747 or 2-0749 cosmetic fluid, which comprise approximately equal parts of a combination of volatile silicones (decamethylcyclopentasiloxane and octamethylcyclotetrasiloxane) and trimethylated silica. This fluid has a viscosity of 200-700 mPa·s (centipoise) as measured by Dow Corning test method CTM 0004A, a specific gravity of 1.000-1.100 at 25°C as measured by Dow Corning test method CTM 0509C, and a refractive index of 1.400-1.410 as measured by Dow Corning test method 0526A. The blend comprises, by weight of the total fluid, approximately 49% of decamethyl cyclopentasiloxane, 1% octamethyl cyclotetrasiloxane and 50% trimethylated silica.

The volatile solvents of this invention generally have a low viscosity ranging from 0.5 to 100, preferably 0.5 to 20, and more preferably 0.5-10 mPa·s (centipoise) at 25°C. Volatile silicones suitable for use in the compositions of this invention include volatile low viscosity silicone fluids such as cyclic silicones having the formula: 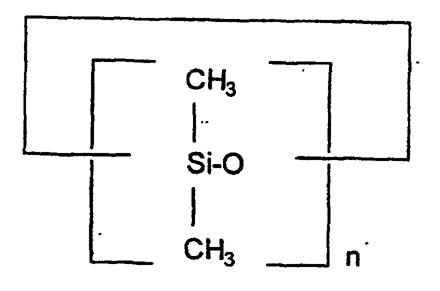 wherein n is from 1 to 7. Volatile linear polydimethylsiloxanes are also suitable and generally have from about 2 to 9 silicon atoms and are of the formula:

(CH₃)₃Si--O-[-Si(CH₃)₂--O-]ₙ--Si(CH₃)₃

wherein n is from 0 to 7. These silicones are available from various sources including Dow Corning Corporation and General Electric. Dow Corning silicones are sold under the tradenames Dow Corning 244, 245, 344, 345 and 200 fluids. These fluids comprise octamethylcyclotetrasiloxane, decamethylcyclopentasiloxane, hexamethyldisiloxane, or mixtures thereof.

The volatile solvent component may comprise straight or branched chain hydrocarbons having from 8 to 20 carbon atoms, more preferably from 10 to 16 carbon atoms. Suitable hydrocarbons are decane, dodecane, tetradecane, tridecane, and C₈₋₂₀ isoparaffins as disclosed in US patent specifications nos. 3,439,088 and 3,818,105. Preferred volatile paraffinic hydrocarbons have a molecular weight of from 160 to 180; a boiling point range of from 105 to 320°C; and a viscosity of less than 20 mPa·s (centipoise) at 25°C. Such paraffinic hydrocarbons are available from Exxon under the ISOPARS registered trademark, and from Permethyl Corporation. Such C₈₋₂₀ paraffinic hydrocarbons such as C₁₂ isoparaffin manufactured by the Permethyl Corporation having the tradename Permethyl 99A (registered trademark) (isododecane) are distributed by Presperse. Various C₁₆ isoparaffins are commercially available, such as isohexadecane (having the tradename Permethyl R (registered trademark)) and are also suitable. The volatile solvent may comprise a mixture of volatile silicone and isoparaffins; a ratio of from 1:20 to 20:1, respectively, is suggested. The volatile solvent preferably ranges 1-40%, more preferably 5-30% by weight of the total composition.

In the preferred embodiment of the invention, from about 5 to about 35% by weight of the total composition comprises a blend of volatile silicones and trimethylated silica, a fluid having the characteristics set forth hereinbefore, and the composition may contain an additional amount of volatile silicone in addition to that found in the blend.

The non-volatile oil, namely dimethicone and/or dimethicone copolyol ranges from 0.1-60%, preferably 0.5-30% by weight of the composition.

Dimethicone and dimethicone copolyol are non-volatile non-fluorinated silicones. Such silicones have a viscosity of 200 to 600,000 mPa·s (centipoise), preferably 350 to 100,000 mPa·s (centipoise) at 25°C), and are available from Dow Corning as the 3225C formulation aid, Dow 190 and 193 fluids, or similar products marketed by Goldschmidt under the ABIL tradename.

The compositions of the invention may be a colour cosmetic such as blusher, liquid makeup, mascara, concealer, and the like.

Creams or lotions are generally water and oil emulsions containing water, humectants, surfactants, preservatives, sunscreens, dry particulate matter, and the like. Generally the ranges of these ingredients are 0.1-80% water, 0.01-10% humectants, 0.01-5% surfactants, 0.001-5% preservatives, and 0.001-10% sunscreens. Suitable emollients, humectants, surfactants, preservatives and sunscreens are as set forth in the C.T.F.A. Cosmetic Ingredient Handbook, First Edition, 1988.

Creams may include humectants, surfactants, thickeners, preservatives and sunscreens, as mentioned above.

The creams and lotions of the invention are particularly good vehicles for sunscreen. In particular, about 0.001-10% by weight of various sunscreen compounds such as PABA (para-amino benzoic acid) and derivatives thereof can be incorporated into the cream or lotion. Because the compositions exhibit superior transfer resistance characteristics, the sunscreens are able to remain on the skin for a longer time period. Especially suitable creams in accordance with the invention are sunscreen creams comprising:
1-30% trimethylated silica
1-40% volatile solvent
0.5-30% non-volatile oil
0.1-70% dry, particulate matter
The dry, particulate matter is selected from titanium dioxide and other powdered materials which provide good sunscreen protection. Usually, titanium dioxide forms the majority of the dry, particulate matter.

Preferably, the compositions of this invention are colour cosmetic compositions such as blush, liquid makeup, and the like.

The particulate matter may be coloured or non-coloured (for example, white) and, in particular, pigments are considered as powders for the purposes of this invention. Suitable powders include bismuth oxychloride, titanated mica, fumed silica, spherical silica, polymethylmethacrylate, micronized Teflon (registered trademark), boron nitride, acrylate polymers, aluminum silicate, aluminium starch octenylsuccinate, bentonite, calcium silicate, cellulose, chalk, corn starch, diatomaceous earth, Fuller's earth, glyceryl starch, hectorite, hydrated silica, kaolin, magnesium aluminum silicate, magnesium carbonate, magnesium hydroxide, magnesium oxide, magnesium silicate, magnesium trisilicate, maltodextrin, montmorillonite, microcrystalline cellulose, rice starch, silica, talc, mica, titanium dioxide, zinc laurate, zinc myristate, zinc neodecanoate, zinc rosinate, zinc stearate, polyethylene, alumina, attapulgite, calcium carbonate, calcium silicate, dextran, kaolin, nylon, silica silylate, silk powder, sericite, soy flour, tin oxide, titanium hydroxide, trimagnesium phosphate, walnut shell powder, or mixtures thereof. The above-mentioned powders may be surface-treated with lecithin, amino acids, mineral oil, silicone oil, or various other agents either alone or in combination, which coat the powder surface and render the particles hydrophobic.

The powder component may also comprise various organic and inorganic pigments. The organic pigments are generally various aromatic types including azo, indigoid, triphenylmethane, anthraquinone, and xanthine dyes which are designated as D&C and FD&C blues, browns, greens, oranges, reds, yellows and the like. Organic pigments generally consist of insoluble metallic salts or certified colour additives, referred to as the Lakes. Inorganic pigments include iron oxides, ultramarine and chromium or chromium hydroxide colours, and mixtures thereof.

The percentage of pigments used in the powder component will depend upon the type of cosmetic being formulated. Blushers and similar cosmetics will contain higher percentages of pigment in the powder phase, usually ranging from 5 to 50% of the total cosmetic composition. Generally the pigment : powder ratio ranges from 1:20 to 20:1.

The compositions of the invention may be mascaras which generally comprise film formers, waxes, emulsifiers, and pigment.

Suitable mascara compositions comprise:
0.1-15% trimethylated silica;
0.1-40% of a volatile solvent having a viscosity of 0.5 to 100 mPa·s (centipoise) at 25°C;
0.1-10% of dimethicone and/or dimethicone copolyol;
0.1-30% of a dry particulate matter having from 5 to 50% pigment in the particle phase;
0.1-20% film former;
0.1-30% wax; and
0.1-10% emulsifier.

Preferably, the volatile solvent comprises a mixture of a volatile silicone and a volatile hydrocarbon, and the dry particulate matter comprises a combination of pigments and non-pigment powders.

Suitable film formers include acacia gum, cellulose derivatives, guar derivatives and all those set forth on pages 68-69 of the C.T.F.A. Cosmetic Ingredient Handbook, First Edition, 1988.

Suitable waxes have a melting point ranging from 35 to 120°C and include natural and synthetic waxes such as bayberry wax, beeswax, candelilla wax, carnauba, ceresin, cetyl esters, hydrogenated jojoba oil, hydrogenated jojoba wax, hydrogenated rice bran wax, japan wax, jojoba butter, jojoba oil, jojoba wax, lanolin wax, microcrystalline wax, mink, montan acid, montan, ouricury, ozokerite, rice bran, shellac, synthetic beeswax and synthetic wax, etc.

Suitable emulsifiers or emulsifying agents are as set forth on pages 90 to 94 of the C.T.F.A. Cosmetic Ingredient Handbook, First Edition, 1988.

The composition of the invention may be makeup compositions or foundations. Makeup generally contains water and pigment in addition to an oil phase. Suitable cosmetic makeup compositions comprise:
0.1-20% trimethylated silica
0.1-40% of a volatile solvent having a viscosity of 0.5 to 100 mPa·s (centipoise) at 25°C,
0.1-25% of dimethicone and/or dimethicone copolyol,
0.1-70% dry particulate matter having a particle size of 0.02 to 100 microns and/or a pigment to powder ratio of 1:20 to 20:1, and
0.1-50% water.

The composition of the invention may also be a blusher. Preferred are blushers comprising:
0.1-20% trimethylated silica,
0.1-30% of a volatile solvent having a viscosity of 0.5 to 100 mPa·s (centipoise) at 25°C.,
0.1-25% of dimethicone and/or dimethicone copolyol
0.1-10% water, and
0.1-70% dry particulate matter having a particle size of 0.02 to 100 microns.

The composition of the invention may also be a concealer, which generally comprises pigment or powder, wax and other ingredients such as humectants, preservatives and others such as those mentioned hereinbefore. A preferred composition of the invention is a concealer comprising:
0.1-15% trimethylated silica,
0.1-40% of a volatile solvent having a viscosity of 0.5 to 100 mPa·s (centipoise) at 25°C,
0.1-35% of dimethicone and/or dimethicone copolyol, and
0.1-40% of a dry particulate matter, optionally having a particle size of 0.02 to 100 microns.

The compositions of the invention provide cosmetics which adhere well to the skin and exhibit improved transfer resistance.

The invention will be further described by reference to the following example which is set forth for the purpose of illustration only.

### EXAMPLE

A makeup composition was made as follows:

| | | w/w% |
|---|---|---|
| 1 | Cyclomethicone/dimethicone copolyol | 20.85 |
| 1 | Sorbitan sesquioleate | 0.05 |
| 1 | Propyl paraben | 0.10 |
| 1 | Titanium dioxide/methicone | 8.00 |
| 1 | Red iron oxide/methicone | 0.47 |
| 1 | Yellow iron oxide/methicone | 1.16 |
| 1 | Black iron oxide/methicone | 0.18 |
| 1 | Mica/dimethicone | 0.98 |
| 2 | Nylon 12/lecithin | 2.00 |
| 2 | Boron nitride | 4.00 |
| 3 | Cyclomethicone | 1.00 |
| 3 | Dimethicone | 1.50 |
| 3 | Dow Corning 2-0747 | 15.00 |
| 3 | Tribehenin | 2.00 |
| 4 | Glyceryl rosinate/C₉₋₁₁ isoparaffin | 5.00 |
| 5 | Water | 30.00 |
| 6 | Methyl paraben | 0.20 |
| 6 | Trisodium EDTA | 0.20 |
| 6 | Butylene glycol | 4.50 |
| 7 | SD alcohol 40-B | 3.00 |

The sequence 1 ingredients were milled in the colloid mill, one after the other until no undispersed white or colour was present. Then sequence 2 ingredients were milled in until dispersed. In the main beaker, sequence 1 and 2 were charged and heated to 55-60°C. Then sequence 3 ingredients were added. When all the tribehenin was melted, the sequence 4 ingredients were added. For the water phase, in a side beaker the sequence 5 ingredients and a pre-mix of sequence 6 ingredients were heated to 50-55°C. Just before emulsification, the sequence 7 ingredients were added to the water phase. The water phase and the oil phase were then emulsified using a homogenizer for 15 minutes. The mixture was cooled using a paddle mixer.

## Claims

1. A cosmetic composition in the form of a water and oil emulsion comprising:
(a) from 0.1 to 60% by weight of trimethylated silica;
(b) from 0.1 to 60% by weight of a volatile solvent having a viscosity of from 0.5 to 100 mPa.s at 25°C;
(c) from 0.1 to 60% by weight of dimethicone and/or dimethicone copolyol; and
(d) from 0.1 to 80% of a cosmetically acceptable carrier;
wherein the volatile solvent comprises a volatile silicone and
wherein at least a portion of the trimethylated silica and the volatile silicone are present as a pre-blended mixture.

2. A composition as claimed in claim 1 wherein the preblended mixture of trimethylated silica and volatile silicone has a viscosity of from 200 to 700 mPa.s.

3. A composition as claimed in any preceding claim, comprising 0.5 to 50% trimethylated silica.

4. A composition as claimed in claim 3, comprising 1 to 30% trimethylated silica.

5. A composition as claimed in any preceding claim, comprising 1 to 40% of the volatile solvent.

6. A composition as claimed in claim 5, comprising 5 to 30% of the volatile solvent.

7. A composition as claimed in any preceding claim comprising 5 to 35% by weight of the preblended mixture of the trimethylated silica and the volatile silicone.

8. A composition as claimed in any preceding claim, comprising 0.5 to 30% of dimethicone and/or dimethicone copolyol.

9. A composition as claimed in any preceding claim wherein the trimethylated silica has an average particle size of from 0.5 to 100 nanometres.

10. A composition as claimed in any preceding claim, wherein the volatile silicone is selected from the group consisting of a cyclic silicone having the formula: 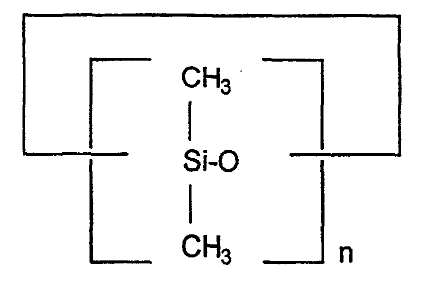 where n is from 1 to 7; a linear silicone having the formula:
(CH₃)₃Si--O- [-Si (CH₃)₂--O-]ₙ--Si(CH₃)₃
where n is from 0 to 7; and mixtures thereof.

11. A composition as claimed in any preceding claim wherein the cosmetically acceptable carrier includes dry particulate matter selected from titanium dioxide, iron oxides, mica, nylon, boron nitride, and mixtures thereof.

12. A composition as claimed in any preceding claim comprising wax.

13. A composition as claimed claim 12, wherein the wax has a melting point of from 35 to 120°C.

14. A composition as claimed in any preceding claim comprising tribehenin.

15. A process for preparing a cosmetic composition in the form of a water and oil emulsion according to any preceding claim, which process comprises bringing
(a) from 0.1 to 60% by weight of trimethylated silica;
(b) from 0.1 to 60% by weight of a volatile solvent having a viscosity of from 0.5 to 100 mPa.s at 25°C; and
(c) from 0.1 to 60% by weight of dimethicone and/or dimethicone copolyol; into intimate physical admixture with from 0.1 to 80% by weight of a cosmetically acceptable carrier therefor;
wherein the volatile solvent comprises a volatile silicone and
wherein at least a portion of the trimethylated silica and the volatile silicone are present as a pre-blended mixture.

16. A cosmetic product comprising a cosmetic composition according to any one of claims 1 to 14 in a suitable container therefor.

17. The use of
(a) from 0.1 to 60% by weight of trimethylated silica;
(b) from 0.1 to 60% by weight of a volatile solvent having a viscosity of from 0.5 to 100 mPa.s at 25°C; and
(c) from 0.1 to 60% by weight of dimethicone and/or dimethicone copolyol;
wherein the volatile solvent comprises a volatile silicone and
wherein at least a portion of the trimethylated silica and the volatile silicone are present as a pre-blended mixture;
in the preparation of a cosmetic composition in the form of a water and oil emulsion for improving skin adhesion and/or transfer resistance of the cosmetic.

## Patentansprüche

1. Kosmetische Zubereitung in Form einer Wasser- und Ö1-Emulsion, umfassend:
(a) von 0,1 bis 60 Gew.-% eines trimethylierten Siliziumoxids;
(b) von 0,1 bis 60 Gew.-% eines flüchtigen Lösungsmittels mit einer Viskosität von 0,5 bis 100 mPa.s bei 25 °C;
(c) von 0,1 bis 60 Gew.-% Dimethicon und/oder eines Dimethiconcopolyols; und
(d) von 0,1 bis 80 % eines kosmetisch annehmbaren Trägers, worin das flüchtige Lösungsmittel ein flüchtiges Silikon umfasst und worin mindestens ein Teil des trimethylierten Siliziumdioxids und des flüchtigen Silikons als vorgemischte Mischung vorhanden sind.

2. Zubereitung gemäß Anspruch 1, worin die vorgemischte Mischung aus trimethyliertem Siliziumdioxid und flüchtigem Silikon eine Viskosität von 200 bis 700 mPa.s aufweist.

3. Zubereitung gemäß einem der vorstehenden Ansprüche, umfassend 0,5 bis 50 % trimethyliertes Siliziumdioxid.

4. Zubereitung gemäß Anspruch 3, umfassend 1 bis 30 % trimethyliertes Siliziumdioxid.

5. Zubereitung gemäß einem der vorstehenden Ansprüche, umfassend 1 bis 40 % des flüchtigen Lösungsmittels.

6. Zubereitung gemäß Anspruch 5, umfassend 5 bis 30 % des flüchtigen Lösungsmittels.

7. Zubereitung gemäß einem der vorstehenden Ansprüche, umfassend 5 bis 35 Gew.-% der vorgemischten Mischung aus trimethyliertem Siliziumdioxid und flüchtigem Silikon.

8. Zubereitung gemäß einem der vorstehenden Ansprüche, umfassend 0,5 bis 30 % Dimethicon und/oder Dimethiconcopolyol.

9. Zubereitung gemäß einem der vorstehenden Ansprüche, worin das trimethylierte Siliziumdioxid eine mittlere Teilchengröße von 0,5 bis 100 nm aufweist.

10. Zubereitung gemäß einem der vorstehenden Ansprüche, worin das flüchtige Silikon ausgewählt ist aus der Gruppe, bestehend aus einem cyclischen Silikon mit der Formel: 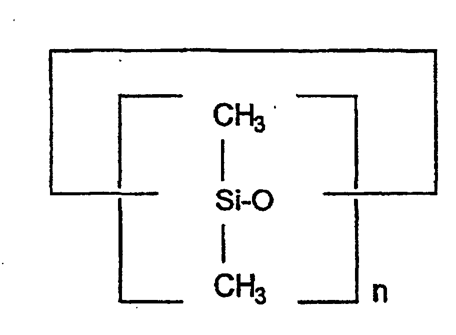 worin n von 1 bis 7 ist, einem linearen Silikon mit der Formel:
(CH₃)₃Si--O-[-Si(CH₃)₂--O-]ₙ--Si(CH₃)₃
worin n von 0 bis 7 ist, und Mischungen derselben.

11. Zubereitung gemäß einem der vorstehenden Ansprüche, worin der kosmetisch annehmbare Träger ein trockenes teilchenförmiges Material umfasst, ausgewählt unter Titandioxid, Eisenoxiden, Glimmer, Nylon, Bornitrid und Mischungen derselben.

12. Zubereitung gemäß einem der vorstehenden Ansprüche, umfassend Wachs.

13. Zubereitung gemäß Anspruch 12, worin das Wachs einen Schmelzpunkt von 35 bis 120 °C aufweist.

14. Zubereitung gemäß einem der vorstehenden Ansprüche, umfassend Tribehenin.

15. Verfahren zur Herstellung einer kosmetischen Zubereitung in Form einer Wasser- und Öl-Emulsion gemäß einem der vorstehenden Ansprüche, welches Verfahren umfasst, Überführen
(a) von 0,1 bis 60 Gew.-% eines trimethylierten Siliziumoxids;
(b) von 0,1 bis 60 Gew.-% eines flüchtigen Lösungsmittels mit einer Viskosität von 0,5 bis 100 mPa.s bei 25 °C; und
(c) von 0,1 bis 60 Gew.-% Dimethicon und/oder eines Dimethiconcopolyols;
in eine innige physikalische Mischung mit 0,1 bis 80 Gew.-% eines hierfür kosmetisch annehmbaren Trägers;
worin das flüchtige Lösungsmittel ein flüchtiges Silikon umfasst und
worin mindestens ein Teil des trimethylierten Siliziumdioxids und des flüchtigen Silikons als vorgemischte Mischung vorhanden sind.

16. Kosmetisches Produkt, umfassend eine kosmetische Zubereitung gemäß einem der Ansprüche 1 bis 14 in einem dafür geeigneten Behälter.

17. Verwendung
(a) von 0,1 bis 60 Gew.-% eines trimethylierten Siliziumoxids;
(b) von 0,1 bis 60 Gew.-% eines flüchtigen Lösungsmittels mit einer Viskosität von 0,5 bis 100 mPa.s bei 25 °C; und
(c) von 0,1 bis 60 Gew.-% Dimethicon und/oder eines Dimethiconcopolyols;
worin das flüchtige Lösungsmittel ein flüchtiges Silikon umfasst; und
worin mindestens ein Teil des trimethylierten Siliziumdioxids und des flüchtigen Silikons als vorgemischte Mischung vorhanden sind;
bei der Herstellung einer kosmetischen Zubereitung in Form einer Wasser- und Öl-Emulsion für die Verbesserung der Hautadhäsion und/oder Transferbeständigkeit des Kosmetikums.

## Revendications

1. Une composition cosmétique sous la forme d'une émulsion eau et huile, comprenant :
(a) de 0,1 à 60 % en poids de silice triméthylée,
(b) de 0,1 à 60 % en poids d'un solvant volatil présentant une viscosité comprise entre 0,5 et 100 mPa/s à 25°C
(c) de 0,1 à 60 % en poids de diméthicone et/ou diméthicone copolyol et
(d) de 0,1 à 80 % d'un support acceptable du point de vue cosmétique,
composition dans laquelle le solvant volatil comprend un silicone volatil et/ou dans laquelle au moins une partie de la silice triméthylée et du silicone volatil sont présents sous la forme d'un pré-mélange.

2. Une composition ainsi que revendiquée dans la revendication 1, dans laquelle le pré-mélange de silice triméthylée et de silicone volatil présente une viscosité comprise entre 200 et 700 mPa/s.

3. Une composition ainsi que revendiquée dans l'une quelconque des revendications précédentes, comprenant de 0,5 à 50 % de silice triméthylée.

4. Une composition ainsi que revendiquée dans la revendication 3, comprenant de 1 à 30 % de silice triméthylée.

5. Une composition ainsi que revendiquée dans l'une quelconque des revendications précédentes, comprenant 1 à 40 % de solvant volatil.

6. Une composition ainsi que revendiquée dans la revendication 5, comprenant de 5 à 30 % de solvant volatil.

7. Une composition ainsi que revendiquée dans l'une quelconque des revendications précédentes, dans laquelle le pré-mélange comprend de 5 à 35 % en poids de silice triméthylée et de silicone volatil.

8. Une composition selon l'une quelconque des revendications précédentes comprenant 0,5 à 30 % de diméthicone et/ou de diméthicone copolyol.

9. Une composition selon l'une quelconque des revendications précédentes, dans laquelle la silice triméthylée présente une dimension particulaire moyenne comprise entre 0,5 et 100 nanomètres.

10. Une composition selon l'une quelconque des revendications précédentes dans laquelle le silicone volatil est choisi dans le groupe comprenant le silicone cyclique présentant la formule : 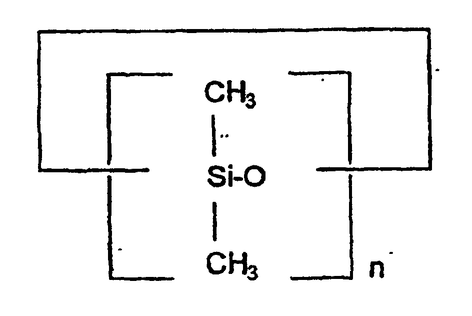 dans laquelle n est compris entre 1 et 7,
et le silicone linéaire présentant la formule :
**(CH**_{**3**}**)**_{**3**}**Si-O-[-Si(CH**_{**3**}**)**_{**2**}**-O-]**_{**n**}**--Si(CH**_{**3**}**)**_{**3**}
dans laquelle n est compris entre 0 et 7 ainsi que leurs mélanges.

11. Une composition ainsi que revendiquée dans l'une quelconque des revendications précédentes dans laquelle le support acceptable du point de vue cosmétique inclut des particules sèches d'un matériau choisi dans le groupe comprenant le dioxyde de titane, les oxydes de fer, le mica, le nylon, le nitrure de bore et leurs mélanges.

12. Une composition ainsi que revendiquée dans l'une quelconque des revendications précédentes comprenant de la cire.

13. Une composition ainsi que revendiquée dans la revendication 12, dans laquelle la cire présente un point de fusion compris entre 35 et 120 °.

14. Une composition ainsi que revendiquée dans l'une quelconque des revendications précédentes comprenant de la tribéhénine.

15. Un procédé de préparation d'une composition cosmétique sous la forme d'une émulsion eau et huile selon l'une quelconque des revendications précédentes, lequel procédé consiste à mettre en contact intime avec 0,1 à 80 % en poids d'un support acceptable du point de vue cosmétique,
(a) de 0,1 à 60% en poids de silice triméthylée
(b) de 0,1 à 60% en poids d'un solvant volatil présentant une viscosité comprise entre 0,5 et 100mPa/s à 25 °C ; et
(c) de 0,1 à 60% en poids de diméthicone et/ou de diméthicone copolyol ;
composition dans laquelle le solvant volatil comprend un silicone volatil et dans lequel une partie au moins de la silice triméthylée et du silicone volatil sont présents sous la forme d'un pré-mélange.

16. Un produit cosmétique comprenant une composition cosmétique selon l'une quelconque des revendications 1 à 14, dans un récipient approprié pour le contenir.

17. L'utilisation de :
(a) 0,1 à 60 % en poids de silice triméthylée ;
(b) 0,1 ) 60 % en poids d'un solvant volatil présentant une viscosité comprise entre 0,5 et 100 mPa/s à 25 °C et
(c) de 0,1 à 60 % en poids de diméthicone et/ou de diméthicone copolyol ;
dans laquelle utilisation le solvant volatil comprend un silicone volatil et dans laquelle au moins une portion de la silice triméthylée et du silicone volatil sont présents sous forme d'un pré-mélange,
pour la préparation d'une composition cosmétique sous la forme d'une émulsion eau et huile destinée à améliorer l'adhésion à la peau et/ou la résistance de transfert du cosmétique.
